(19) Europäisches Patentamt
European Patent Office
Office européen des brevets

(11) Publication number : **0 659 750 A1**

# (12) EUROPEAN PATENT APPLICATION

(21) Application number : **94309681.8**

(51) Int. Cl.$^6$ : **C07D 401/14**, C08K 5/3492

(22) Date of filing : **22.12.94**

(30) Priority : **24.12.93 JP 329084/93**
**01.07.94 JP 151221/94**
**01.07.94 JP 151220/94**

(43) Date of publication of application :
**28.06.95 Bulletin 95/26**

(84) Designated Contracting States :
**BE CH DE ES FR GB IT LI NL**

(71) Applicant : **SUMITOMO CHEMICAL COMPANY LIMITED**
**5-33, Kitahama 4-chome,**
**Chuo-ku**
**Osaka-shi, Osaka 541 (JP)**

(72) Inventor : **Kimura, Kenji**
**2-29-7 Oike**
**Ibaraki-shi, Osaka (JP)**
Inventor : **Tanaka, Shinya**
**2-11-7-205 Sonehigashino-cho**
**Toyonaka-shi, Osaka (JP)**
Inventor : **Sasaki, Manji**
**31-15-203 Kawarabayashi-cho**
**Nishinomiya-shi, Hyogo (JP)**

(74) Representative : **Cresswell, Thomas Anthony**
**J.A. KEMP & CO.**
**14 South Square**
**Gray's Inn**
**London WC1R 5LX (GB)**

## (54) A process for the preparation of polyaminotriazines.

(57) A process for preparing polyaminotriazines represented by the following formula (I) :

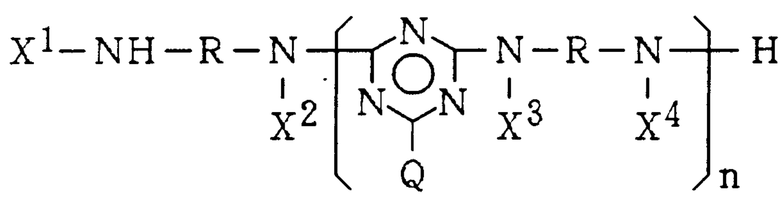

(I)

wherein n is a number from 2 to 20 ;
$X^1$,$X^2$,$X^3$ and $X^4$ is hydrogen or piperidyl ;
R is alkylene or a divalent cycloaliphatic group ; and
Q is $-OR^3$, $-NHR^4$ or $-NR^4R^5$ wherein $R^3$, $R^4$ and $R^5$ is alkyl etc., which comprises ;
reduction alkylamination of a tetramethylpiperidone with a diamine represented by the following formula (IIb),

$$NH_2\text{- R -}NH_2 \qquad \text{(IIb)}$$

wherein R is as defined above,
in the presence of hydrogenating catalyst ; removing off the catalyst after the completion of the reaction to obtain a crude product (A) ;
and then, without refining the crude product (A), carrying out a polycondensation reaction between the crude product (A) and a dichlorotriazine in an aromatic solvent in the presence of an inorganic base.

EP 0 659 750 A1

The present invention relates to an improved process for the preparation of polyaminotriazines useful as light stabilizers for an organic material, particularly for synthetic resins. In more detail, it relates to an improved process for the preparation of polyaminotriazines represented by the following formula (I):

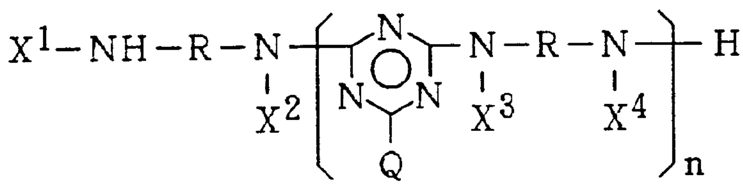
(I)

wherein n is a number from 2 to 20;
$X^1$, $X^2$, $X^3$ and $X^4$, which are same or different, are each hydrogen or piperidyl represented by the following formula(Ia),

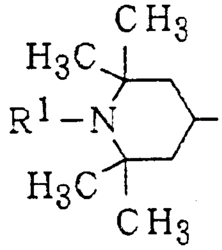
(Ia)

wherein $R^1$ is a hydrogen atom, $C_1$ to $C_{12}$ alkyl, $C_1$ to $C_{18}$ alkoxy, $C_3$ to $C_8$ alkenyl, $C_7$ to $C_{11}$ arylalkyl or $C_3$ to $C_5$ alkenyloxy, provided that 75 mole% or more of $X^1$,$X^2$,$X^3$ and $X^4$ is piperidyl of the formula (Ia);
R is $C_2$ to $C_{12}$ alkylene which can be interrupted by -O- or - $NR^2$- wherein $R^2$ is hydrogen, $C_1$ to $C_{12}$ alkyl, $C_3$ to $C_{12}$ cycloalkyl or piperidyl of the formula (Ia),
or R is a divalent $C_6$ to $C_{15}$ cycloaliphatic group; and
Q is $-OR^3$, $-NHR^4$ or $-NR^4R^5$.
wherein $R^3$ is $C_1$ to $C_{12}$ alkyl, $C_5$ to $C_{12}$ cycloalkyl, benzyl, phenyl, tolyl or piperidyl of the formula (Ia),
$R^4$ is $C_1$ to $C_{12}$ alkyl, $C_3$ to $C_{12}$ alkoxyalkyl, $C_4$ to $C_{12}$ N,N-dialkylaminoalkyl, $C_3$ to $C_5$ alkenyl, phenyl, benzyl, $C_5$ to $C_{12}$ cycloalkyl such as cyclohexyl, tolyl or piperidyl of the formula (Ia), and $R^5$ is $C_1$ to $C_{12}$ alkyl or $C_5$ to $C_{12}$ cycloalkyl, or $R^4$ and $R^5$, together with the N atom to which they are bonded, form 5- or 6- membered heterocyclic ring.

It is known that organic materials, such as synthetic polymers i.e. polyethylene, polypropylene or the like, are liable to undergo a deterioration of their properties, such as softening, embrittlement and discoloration, when they are exposed to light. In order to prevent such deterioration, various stabilizers have been proposed and polyaminotriazines of the formula (I) has been known as one of the stabilizers.

As described in JP-A-52-71486 ("JP-A-" means " Japanese unexamined patent publication", i.e. "Kokai") or JP-A-58-201820, etc, the polyaminotriazines of the formula (I) have been usually prepared by polycondensation reaction of a monopiperidylamine or a dipiperidylamine represented by the following formula (II);

$$X\text{-}NH\text{-}R\text{-}NH\text{-}X^5 \qquad (II)$$

wherein R is as defined above, X is piperidyl of the formula (Ia) and $X^5$ is hydrogen or piperidyl of the formula (Ia), with a dichlorotriazine represented by the following formula (III);

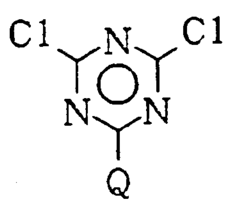
(III)

wherein Q is as defined above .

The diamine of the formula (II) has been prepared by reacting a tetramethylpiperidone represented by the

following formula (IIa);

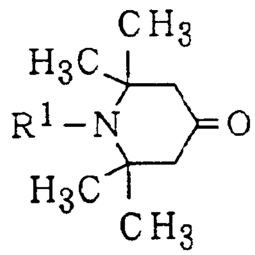

(IIa)

wherein $R^1$ is as defined above,
with a diamine represented by the following formula (IIb);

$NH_2$-R-$NH_2$ (IIb)

wherein R is as defined above (JP-B-58-11454 : "JP-B-" means " Japanese examined patent publication", i.e. "Kokoku", JP-A-64-50858, JP-A-5-86029, etc.).

Hitherto, the preparation of the polyaminotriazines of the formula (I) from the diamine of the formula (II) and the dichlorotriazine of the formula (III) have been carried out independently from the preparation of the diamine of the formula (II). That is, the dipiperidylamine of the formula (II) prepared by a reaction between a tetramethylpiperidone of the formula (IIa) and a diamine of the formula (IIb) has been isolated from the resulting reaction mixture before being used for the preparation of the polyaminotriazines of the formula (I), because, without the isolation of the dipiperidylamine of the formula (II), it has been thought that the polyaminotriazines of the formula (I) having sufficient properties as stabilizers could not been obtained. However, according to the above-process, high yield of the polyaminotriazine of the formula (I) based on the diamine of formula (IIb) could not be achieved.

The object of the present invention is to provide an effective process according to which polyaminotriazines of the formula (I) having good properties as stabilizers can be obtained at high yield.

Inventors of the present invention have conducted studies to develop an effective process for preparing polyaminotriazines and, as the result, found that polyaminotriazines of the formula (I) having good properties as stabilizers can be prepared at high yield by a polycondensation reaction of a dichlorotriazine of the formula (III) with the reaction product of a reduction alkylamination between a tetramethylpiperidone of the formula (IIa) and a diamine of the formula (IIb) without refining the reaction product, i.e. isolating the diamine of the formula (II) from the resulting reaction mixture.

The present invention provides a process for preparing polyaminotriazines of the formula (I) which comprises;
carrying out a reduction alkylamination of a tetramethylpiperi done of the formula (IIa) with a diamine of the formula (IIb) in the presence of a hydrogenating catalyst;
removing off the catalyst after the completion of the reaction to obtain a crude product (herein after, referred to as "crude product (A)" );
and then, without refining the crude product (A), carrying out a polycondensation reaction between the crude product (A) and the dichlorotriazine of the formula (III) in an aromatic solvent in the presence of an inorganic base.

As an example of the polyaminotriazines which can be produced according to the present invention, a polyaminotriazines of the formula (I) wherein $R^1$ is hydrogen or methyl, R is straight chain or branched chain alkylene having 2 to 12 carbon atoms and Q is -$NHR^4$ or -$NR^4R^5$ can be mentioned. As a more concrete example, a polyaminotriazines of the formula (I) wherein $R^1$ is hydrogen, R is hexamethylene Q is 1,1,3,3-tetramethylbutylamino can be mentioned.

Among tetramethylpiperidones of the formula (IIa), those wherein $R^1$ is hydrogen or methyl are particularly preferred. When $R^1$ in the formula (Ia) or (IIa) is arylalkyl, a typical example of $R^1$ is phenyl alkyl.

Among diamines of the formula (IIb), those wherein R is straight or branched chain alkylene having 2 - 12 carbon atoms are preferred, more preferably R is straight chain alkylene having 2 - 8 carbon atoms and particularly preferably R is ethylene or hexamethylene. The diamine of the formula (IIb) may contain water: diamine containing up to about 20 weight% of water based on the diamine can be used.

The divalent cycloaliphatic groups represented by R in the formula (IIb) are groups having cycloalkane ring which include, for example, cycloalkylene such as cyclohexylene, methylene dicycloalkylene such as methylene dicyclohexylene and cycloalkylene methylene such as cyclohexylene methylene which may be optionally substituted by an alkyl as long as the number of the carbon atoms is 12 or less.

When Q is $-NHR^4$, $R^4$ is preferably a straight or branched chain alkyl having 4 - 8 carbon atoms. When Q is - $NR^4R^5$ which forms 5- or 6-membered heterocyclic ring, examples of Q include 1-pyrrolidinyl, 1-imidazolidinyl, piperidino, 1-piperazinyl and morpholino, among which morpholino is preferred. When Q is $-NHR^4$, 1,1,3,3,-tetramethylbutylamino is also preferred as Q.

Tetramethylpiperidones of the formula (IIa) and diamines of the formula (IIb) are produced on a commercial basis and are available easily.

A tetramethylpiperidone of the formula (IIa) and a diamine of the formula (IIb) are subjected to a reduction alkylamination in the presence of a hydrogenating catalyst.

As the examples of the hydrogenation catalyst used in the reduction alkylamination, platinum, nickel, cobalt and palladium can be mentioned. Among them, platinum or palladium supported on carbon (hereinafter described as platinum/carbon or palladium/carbon, respectively) and Raney nickel are preferred.

When the hydrogenation catalyst used in the reduction alkylamination is platinum or palladium, the amount of the catalyst is from 0.001 to 0.04 weight% based on the tetramethylpiperidone of the formula (IIa) and the reaction temperature is from 40 to 140 °C. When the hydrogenation catalyst is Raney nickel, the amount of the catalyst is from 0.1 to 30 weight% based on the tetramethylpiperidone of the formula (IIa) and the reaction temperature is from 100 to 180 °C.

The amount of the tetramethylpiperidone of the formula (IIa) is preferably 1.5 moles or more and less than 2.1 moles and particularly preferred is from 1.7 to 1.95 moles based on the amount of the diamine of the formula (IIb). If 2.1 moles or more of a tetramethylpiperidone of the formula (IIa) is used in the reaction and the resulting product is used for the polycondensation with a dichlorotriazine of the formula (III) without refining the resulting product, the polyaminotriazine thus obtained is often colored in red-brown. On the other hand, if the amount of the tetramethylpiperidone of the formula (IIa) is too little such as less than 1.5 moles, stabilizing effect of the polyaminotriazines of the formula (I) becomes insufficient. Using 1.5 moles or more of the tetramethylpiperidone of the formula (IIa) per 1.0 mole of the diamine of the formula (IIb) in the reduction alkylamination, polyaminotriazines of the formula (I) wherein 75mole% or more of $X^1$,$X^2$,$X^3$ and $X^4$ is piperidyl of the formula (Ia) can be obtained.

The reduction alkylamination of a tetramethylpiperid one of the formula (IIa) with a diamine of the formula (IIb) can be carried out according to a known manner. For example, this reaction can be carried out in the presence of platinum, palladium or Raney nickel in methanol, ethanol, propanol or a mixture of the alcohol and water. Carrying out the reaction according to this manner, small amount of by-products other than monopiperidyl compounds is formed and the resulting crude product can be used for the polycondensation with a dichlorotriazine of the formula (III) only after filtering off the catalyst and distilling off the solvent. Therefore, the above-mentioned manner is preferred. An example of the preferred manner is described in JP-A-58-11454, in which the reaction is carried out in methanol in the presence of platinum/carbon or palladium/carbon at 55 - 75°C under a hydrogen pressure of 9 - 10 atm. for 4 - 5 hours.

Another manner which is similar to that described in JP-A-5-86029 can also be employed. According to the manner, at first, a reaction between a tetramethylpiperidone of the formula (IIa) and a diamine of the formula (IIb) is carried out at 50 - 100 °C in the absence of solvent while distilling off water in the reaction mixture under a reduced pressure such as 1 - 135 Torr, and then a reduction alkylamination of the resulting product with hydrogen is carried out in the presence of platinum, palladium or Raney nickel to obtain a crude product. Since the crude product thus obtained can be used for the polycondensation only after filtering off the catalyst, this manner is also very preferred. As a more concrete example of this preferred manner, following manner can be mentioned:

the reaction between a tetramethylpiperidone of the formula (IIa) and a diamine of the formula (IIb) is carried out, first, at 50 - 60°C for 1 hour without using solvent and, then, at 60°C 30 Torr for 3 hour, while distilling off water and, thereafter, a reduction alkylamination with hydrogen is carried out in the presence of platinum/carbon or palladium/carbon at 90 °C at a hydrogen pressure of 5 atm. for 4 hours or in the presence of decanted Raney nickel at 100 °C at a hydrogen pressure of 100 atm. for 4 hours.

The crude product mainly contains the diamine of the formula (II). However, it also contains less than 1 weight% of by-products such as N-(2-propyl)-N'-(2,2,6,6-tetramethyl-4-piperidyl)hexamethylenediamine even if using tetramethylpiperi done of the formula (IIa) having a high purity of 99 weight% or more which was obtained by refining such as distillation, recrystalization or the like. However, contamination of such very small amount of the by-products in polyaminotriazines of the formula (I) does not have any effects on the properties of the polyaminotriazines.

The dichlorotriazines of the formula (III) can be prepared according to a known process such as a process described in JP-A-52-71486 in which the dichlorotriazines is prepared by reacting cyanuric trichloride and a monofunctional compound of the formula -QH, wherein Q is as mentioned above.

The resulting reaction solution can be used for the process of the present invention without isolating the

dichlorotriazines of the formula (III). Although using the resulting reaction solution without isolating the dichlorotriazines is preferred, the dichlorotriazines of the formula (III) which was isolated from the resulting reaction solution in a known method such as recrystallization can also be used in the present invention.

Prefered examples of the dichlorotriazines of the formula (III) include 2,4-dichloro-6-(1,1,3,3-tetramethylbutyl amino)-1,3,5-triazine and 2,4-dichloro-6-morpholino-1,3,5-triazine.

The polycondensation degree of the polyaminotriazine s of the formula (I) can be varied depending on the amount of the dichlorotriazine of the formula (III) used in the polycondensation reaction. The preferred amount of the dichlorotriazine of the formula (III) is usually 0.75 - about 1 mole, particularly preferably 0.8 - 0.9 mole, based on 1 mole of the diamine of the formula (IIb). If the amount is less than 0.75 mole, the polyaminotriazines having low polycondensation degree (such as that wherein n in the formula (I) is less than 5) is obtained. On the other hand, if the amount exceeds about 1 mole, the polycondensation does not proceed to completion.

In the polycondensation reaction, a solid inorganic base or aqueous solution thereof is used. If an organic base such as triethylamine is used, the polyaminotriazines having relatively low polycondensation degree (n in the formula (I) is less than 6) is obtained and the yield of the polycondensation is low.

As preferable examples of the inorganic base, alkali metal hydroxide such as sodium hydroxide or potassium hydroxide and alkali metal carbonate such as sodium carbonate or potassium carbonate can be mentioned. Sodium hydroxide or potassium hydroxide is particularly preferred.

Preferred amount of the inorganic base used in the polycondensation reaction is 2 - 3 equivalents, particularly preferably 2.2 - 2.5 equivalents, per 1 equivalent of the dichlorotriazine of the formula (III). If the amount is less than 2 equivalents, the polycondensation hardly proceeds to completion. The amount exceeding 3 equivalents is disadvantageous from the economical view.

Aromatic solvents being water-immiscible and inert to the polycondensation reaction can be used for the reaction. As examples of the solvent, xylene, ethylbenzene, toluene, o-dichlorobenzene and mesitylene can be mentioned. If a solvent having the boiling point higher than 170 °C under atomospheric pressure such as o-dichlorobenzene is used, high temperature and/or high vacuum is required in order to remove the solvent from the reaction mixture. Therefore, solvent having relatively low boiling point, for example solvent having the boiling point of about 100 - 160°C under atomospheric pressure such as xylene, ethylbenzene, toluene or a mixture thereof is preferred. Among them, xylene, ethylbenzene or a mixture of xylene and ethylbenzene is particularly preferred. For smooth operation, 0.1 weight part or more of solvent per 1 weight part of the crude product (A) is usually required.

Though the polycondensation reaction can proceed at a temperature of 80 °C or higher, the range of the reaction temperature should be from 145 to 220 °C and preferably from 155 to 190 °C when the reaction is carried out under an elevated pressure. When the reaction is carried out under atmospheric pressure, the preferable range of the reaction temperature is from 145 to 190 °C as explained below. If the reaction temperature is lower than 145 °C, long reaction time is required and a drop of the polycondensation degree, an increase of by-products and/or a drop of yield are often caused. If the reaction temperature exceeds 220°C , the starting materials and the reaction products are often decomposed.

The polycondensation reaction can be carried out in various manner. For example, the inorganic base, crude product (A) and the dichlorotriazine of the formula (III) were mixed at room temperature and, then, the reaction temperature is raised to a specific temperature which is kept thereafter to carry out the reaction. However, in order to suppress by-products and to improve the yield, the dichlorotriazine of the formula (III) is preferably added to solution of crude product (A) dispersing an inorganic base at the specific reaction temperature. As the dichlorotriazine of the formula (III), a dichlorotriazine which was isolated from the reaction mixture obtained by the reaction between cyanuric trichloride and a monofunctional compound of the formula -QH and was dissolved in an appropriate solvent can be used for the polycondensation reaction. However, it is preferred that a reaction mixture is used without isolating resulting dichlorotriazine. The polycondensation reaction can be carried out either under an elevated pressure or under atmospheric pressure.

Under an elevated pressure by using an autoclave, for example, a reaction temperature suitable for the polycondensation can be easily achieved regardless of the amount of the aromatic solvent. In this case, it is preferred to use the inorganic base as a concentrated aqueous solution. For example, the polyaminotriazines of the formula (I) can be prepared by dropwise and slowly adding a solution of the dichlorotriazine of the formula (III) in an aromatic solvent to a mixture of the crude product (A) and 50% aqueous solution of sodium hydroxide or potassium hydroxide in an autoclave while keeping the temperature in a range from 145 to 220°C to carry out the polycondensation reaction under an elevated pressure.

If the polycondensation reaction is carried out under atmospheric pressure, a reaction temperature suitable for the reaction can be kept by distilling off water such as water generated in the reaction, and, if necessary, a part of the solvent from the reaction system, while adding solution of the dichlorotriazine of the formula (III). However, in this case, in order to achieve the reaction temperature higher than 190 °C, usually, very small

amount of the solvent must be used and the workability is not good. On the other hand, the higher reaction temperature is preferred in order to achieve high yield and high polycondensation degree. Therefore, under atmospheric pressure, it is preferred to carry out the polycondensation reaction at a temperature of 145 - 190°C, particularly preferably 155 - 180 °C, using the solvent in an amount of 0.1 - 1 weight part, particularly preferably 0.2 - 0.8 weight part per 1 weight part of the crude product (A).

If the polycondensation reaction is carried out under atmospheric pressure, the inorganic base should be used in a solid form. Preferable examples of the inorganic base include powder or flake of sodium hydroxide or potassium hydroxide. Particularly, sodium hydroxide is preferred.

The suitable amount of the solvent, i.e. 0.1 - 1 weight part based on 1 weight part of the crude product (A), can be achieved at the completion of the addition of the dichlorotriazine of the formula (III) by adding the dichlorotriazine slowly and dropwise over long period and by distilling off water and, if necessary, a part of the solvent from the reaction system. The suitable amount, i.e. 0.1 - 1 weight part per 1 weight part of the crude product(A), may be achieved until start of the main stage of the process to obtain the preferable results. Therefore, a solution of the dichlorotriazine of the formula (III) containing more than 1 weight part of the solvent per 1 weight part of the crude product (A) can be used to obtain the preferable results as long as the condition of 0.1 - 1 weight part is achieved by distilling off a part of solvent.

According to the present invention, polyaminotriazin es of the formula (I) having sufficient properties as stabilizers can be obtained at a high yield.

The present invention also provides a process for preparing polyaminotriazines of the formula (I) which comprises
reacting a diamine of the formula (II) with a dichlorotriazine of the formula (III) in a mole ratio (III)/(II) of 0.83 to 0.98, provided that more than 80 mole% of the diamine of the formula (II) is that wherein $X^5$ is piperidyl of the formula (Ia),
in 0.1 - 1 weight part of a water-immiscible aromatic solvent per 1 part of diamine of the formula (II),
using an inorganic base in a solid form, under atmospheric pressure at a temperature higher than the boiling point of the solvent while azeotropically distilling off water generated in the reaction. (Hereinafter, this process is referred to as Process A)

To put it simply, the characteristics of Process A reside in carrying out the reaction under atmospheric pressure in the presence of small amount of aromatic solvent at a temperature higher than the boiling point of the aromatic solvent while azeotropically distilling off water generated in the reaction.

According to JP-A-52-71486, the polyaminotriazines are prepared by polycondensation of a dichlorotriazine and a monopiperidylamine or dipiperidylamine of the formula (II) in an inert solvent at the boiling point of the solvent or a lower temperature in the presence of an inorganic or organic base.

In more detail, this reaction is carried out mainly in refluxing toluene and sodium hydroxide is used as the base. Since the reaction mixture contains water generated from the reaction, the reflux is usually took place at a temperature about 10 °C lower than the boiling point of toluene. The polyaminotriazine was obtained from the reaction mixture by filtering off the by-produced sodium chloride and by evaporating off the solvent.

This process, however, has disadvantages that the reaction hardly proceeds to completion, and that sparingly soluble by-products having high melting point are formed. Because these by-products are sparingly soluble to synthetic resins to be stabilized by the polyaminotriazines and they deteriorate the value of the synthetic resin products, the polyaminotriazines containing the by-products are undesirable as stabilizers. Therefore, the by-products need to be separated off from the resulting reaction mixture by filtration. However, the filtration causes loss in the yield. After having separated off the by-products, the yield is thus only about 70 - 75%.

This process has another disadvantages that only polycondensates having relatively low polycondensation degree of less than 6 can be obtained. As a stabilizer for a synthetic resin used in the open air or the like, a polyaminotriazine having polycondensation degree of 6 or more is desired, and that having a polycondensation degree of 7 to 11 is more preferred.

In JP-A-58-201820, an improved process for the preparation of the polyaminotriazines is described. According to JP-A-58-201820, the polyaminotriazines are prepared by a polycondensation of a dichlorotriazine, which is obtained by a substitution of a chlorine atom of cyanuric chloride with a radical such as amine, with a diamine in a water-immiscible inert solvent at a temperature of from 140 to 220°C under an elevated pressure by using aqueous solution of an inorganic base.

In an example of this process, this reaction is carried out in xylene/water two-phase system under an elevated pressure at a temperature of about 185 °C using a concentrated aqueous solution of sodium hydroxide as the base. The reaction mixture is subjected to phase-separation, washing and oil-water separation to obtain xylene phase, which is, then, filtered for filtering off the by-products. The polyaminotriazine is obtained from the resulting filtrate by evaporating off the solvent. According to the process, the polyaminotriazine can be obtained at a high yield of 93 - 96% based on the amount of the diamine of formula (II), but this process still

has following disadvantages.

One disadvantage is that a special and high-priced material which is alkaline- and heat-resisting must be used for the reaction vessel because the reaction is carried out at high temperature, under alkaline conditions, under high pressure in the presence of water. These conditions are so severe that even SUS 316L, which has been widely used for pressure reaction vessels, is eroded. Therefore, even SUS 316L cannot be used for the reaction vessel.

Another disadvantage is that the polyaminotriazine having low polycondensation degree is liable to be obtained because the reaction components may come in contact with water since this reaction is carried out in xylene/water two-phase system and the chlorotriazine intermediate of this reaction is hydrolyzed at a high temperature of about 185 °C when it comes in contact with water.

Inventors of the present invention have conducted studies to solve the above-disadvantages and, as the result, found specific conditions to obtain polyaminotriazines of the formula (I) effectively and attained Process A.

According to Process A, forming of by-products and hydrolysis of the intermediate are suppressed and high yield of the polyaminotriazine is achieved and a reaction vessel made of SUS 316L can be used.

As mentioned above, the crude product (A) can be used for Process A as the diamine of the formula (II). The advantages of this process can also be achieved, even when using the diamine of the formula (II) which was isolated by a known method such as distillation or recrystalization. If isolated diamine is used, less colored polyaminotriazines is obtained. Therefore, in order to reduce the color of the product, using an isolated diamine is preferred. Diamines of the formula (II) prepared by mixing an isolated diamine wherein $X^5$ is hydrogen and an isolated diamine wherein $X^5$ is piperidyl at desired proportion can also be used and the advantages of Process A can be achieved.

For Process A, diamines of the formula (II) wherein R is straight or branched chain alkylene having 2 - 12 carbon atoms are preferably used, more preferably R is straight chain alkylene having 2 - 8 carbon atoms and particularly preferably R is ethylene or hexamethylenediamine. $R^1$ in the formula (Ia) is preferably hydrogen or methyl.

In order to prepare polyaminotriazines of the formula (I) wherein 75 mole% or more of $X^1$,$X^2$,$X^3$ and $X^4$ is piperidyl of the formula (Ia), 80 mole % or more of the diamine of the formula (II) must be that wherein $X^5$ is the piperidyl of the formula (Ia). If the ratio is less than 80 polyaminotriazines having inferior properties as a stabilizer for synthetic resins are liable to be produced.

Examples of preferable diamine of the formula (II) usable in Process A include:
N,N'-bis(2,2,6,6-tetramethyl-4-piperidyl)hexamethylenediamine, N,N'-bis(1,2,2,6,6-pentamethyl-4-piperidyl)hexamethylenediamin e.
a mixture of N-(2,2,6,6-tetramethyl-4-piperidyl) hexamethylenediamine and N,N'-bis(2,2,6,6-tetramethyl-4-piperidyl)hexamethylenediamine, and
a mixture of N-(1,2,2,6,6-pentamethyl-4-piperidyl) hexamethylenediamine and N,N'-bis(1,2,2,6,6-pentamethyl-4-piperidyl)hexamethylenediamine.

In this invention atmospheric pressure includes such a pressure which is easily attained without a special manipulation such as compressing or decompressing.

In order to achieve the advantages of Process A, the ratio of the dichlorotriazine of the formula (III) to the diamine of the formula (II) should be from 0.83 to 0.98, the reaction temperature must be higher than the boiling point of the aromatic solvent, and the inorganic base must be used in a solid form. If an aqueous solution of the inorganic base is used, a reaction temperature higher than the boiling point of the aromatic solvent can hardly be achieved.

As an example of the preferable manner of Process A, following manner can be mentioned:
polycondensation reaction is carried out by slowly adding a solution of the dichlorotriazine of the formula (III) in an aromatic solvent to a mixture of the diamine of the formula (II) and sodium hydroxide or potassium hydroxide which is kept at a temperature higher than the boiling point of the solvent while distilling off water generated in the reaction and a part of the solvent.

After the completion of the polycondensation, the reaction mixture is washed with water, dried and filtered. The filtration can be accelerated by adding a filter aid, such as cellulose, diatomaceous earth and fuller's earth. By evaporating solvent from the filtrate, the polyaminotriazines of the formula (I) is obtained in a solid mass, which is crushed, if necessary.

Examples of the polyaminotriazines of the formula (I) which can be obtained effectively according to the present invention include:

a polycondensate of 2,4-dichloro-6-(1,1,3,3-tetramethylbutylamino)-1,3,5-triazine with a mixture of N-(2,2,6,6-tetramethyl-4-piperidyl)hexamethylenediamine and N,N'-bis(2,2,6,6-tetramethyl-4-piperidyl)hexamethylenediamine,

a polycondensate of 2,4-dichloro-6-morpholino-1,3,5-triazine with a mixture of N-(2,2,6,6-tetramethyl-4-piperidyl) hexamethylenediamine and N,N'-bis(2,2,6,6-tetramethyl-4-piperidyl)hexamethylenediamine,

a polycondensate of 2,4-dichloro-6-(1,1,3,3-tetramethylbutylamino)-1,3,5-triazine with a mixture of N-(1,2,2,6,6-pentamethyl-4-piperidyl)hexamethylenediamine and N,N'-bis(1,2,2,6,6-pentamethyl-4-piperidyl)hexamethylenediamine,

a polycondensate of 2,4-dichloro-6-morpholino)-1,3,5-triazine with a mixture of N-(1,2,2,6,6-pentamethyl-4-piperidyl)hexamethylenediamine and N,N'-bis(1,2,2,6,6-pentamethyl-4-piperidyl)hexamethylenediamine.

If isolated diamine is used, following can also be mentioned as preferable examples:

a polycondensate of 2,4-dichloro-6-(1,1,3,3-tetramethylbutylamino)-1,3,5-triazine with N,N'-bis(2,2,6,6-tetramethyl-4-piperidyl)hexamethylenediamine,

a polycondensate of 2,4-dichloro-6-morpholino-1,3,5-triazine with N,N'-bis(2,2,6,6-tetramethyl-4-piperidyl) hexamethylenediamine,

a polycondensate of 2,4-dichloro-6-(1,1,3,3-tetramethylbutylamino)-1,3,5-triazine with N,N'-bis(1,2,2,6,6-pentamethyl-4-piperidyl)hexamethylenediamine, and

a polycondensate of 2,4-dichloro-6-morpholino)-1,3,5-triazine with N,N'-bis(1,2,2,6,6-pentamethyl-4-piperidyl) hexamethylenediamine.

The following examples illustrate the present invention in more detail. They are illustrative and should not be interpreted as to limit the present invention. In the examples, "%" means "weight %", unless otherwise mentioned.

Example 1

(a) Preparation of intermediate

Into an autoclave, 94.6 g (0.609 mole) of 2,2,6,6-tetramethyl-4-piperidone, 37.3 g (0.0.321ole) of hexamethylenediamine, 200 g of methanol and 0.55 g of 5 % platinum/carbon were charged and the mixture was kept at 65°C, hydrogen pressure of 9 - 10 atom for 3 hours and thereafter it was kept at 70 °C for 1 hour. (The molar ratio of 2,2,6,6-tetramethyl-4-piperidone to hexamethylenediamine is 1.9/1.) After completion of the reaction, catalyst was filtered off and methanol was distilled off under reduced pressure to obtain 119.7 g of a concentrated mass. According to an analysis with gas-chromatography, the mass thus obtained contained 94.4 % of N,N'-bis(2,2,6,6-tetramethyl-4-piperidyl) hexamethylenediamine and 5.4 % of N-(2,2,6,6-tetramethyl-4-piperidyl)hexamethylenediamine.

(b) Preparation of dichlorotriazine

Into flask, 20 g of water, 52.3 g (0.284 mole) of cyanuric chloride and 130 g of mixed xylene having the boiling point of 138 - 141 °C were charged and 37.5 g (0.290 mole) of 1,1,3,3-tetramethylbutylamine was slowly and dropwise added into the mixture while keeping the temperature at 8 - 10 °C. Then, 57.9 g of 20 % aqueous hydroxide solution was slowly and dropwise added into the mixture while keeping the temperature at 8 - 10 °C . After completion of the reaction, oil and water phase were separated and the water phase was removed to obtain a xylene solution of 2,4-dichloro-6-(1,1,3,3-tetramethylbutylamino)-1,3,5-triazine.

(c) Preparation of polyaminotriazine

Into 500 ml four necked flask equipped with a dean-stark trap, whole of the concentrated mass obtained in (a) and 28.4 g (0.71 mole) of sodium hydroxide powder were charged, and inner temperature was raised to 160 °C. Then, into the mixture, the xylene solution of 2,4-dichloro-6-(1,1,3,3-tetramethylbutylamino)-1,3,5-triazine obtained in (b) was dropwise added over 4 hours. After the dropwise addition started, reflux of water generated in the reaction started, the refluxing water being distilled off, and, after a while, reflux of xylene also started. Keeping the dropwise addition for a while, the reaction temperature started lowering. In order to keep the reaction temperature at 160°C or higher, a part of the refluxing xylene was distilled off. At the end of addition of the solution, total amount of the xylene distilled off was 88 g, which means that, at the end of the addition, xylene content in the mixture was 0.35 weight part per 1 weight part of the concentrated mass. After the end of the addition of the solution, the reaction temperature was kept at 160 °C for 5 hours while distilling off water generated in the reaction. After completion of the reaction, water was added to the reaction mass in order to dissolve sodium chloride generated in the reaction and removed off. Resulting xylene solution was filtered to remove by-products and concentrated. Then, the resulting product was cooled to obtain a solid resin, which was crushed.

Yield 92 %

(From Example 1 to 7, Yields are based on diamine used for the reaction in (a).)

Number average molecular weight 4900

n = 7.5

Example 2

(a) Preparation of intermediate

Example 1(a) was repeated, except that reaction condition of "at 65 °C for 3 hours" was replaced by "at 60°C for 3.5 hours" to obtain 120.1 g of concentrated mass. The mass thus obtained contained 87.0 % of N,N'-bis(2,2,6,6-tetramethyl-4-piperidyl)hexamethylenediamine and 12.1 % of N-(2,2,6,6-tetramethyl-4-piperidyl)hexamethylenediamine.

(b) Preparation of dichlorotriazine

Example 1(b) was repeated to obtain a xylene solution of 2,4-dichloro-6-(1,1,3,3-tetramethylbutylamino)-1,3,5-triazine.

(c) Preparation of polyaminotriazine

Example 1(c) was repeated, except that the concentrated mass and xylene solution of 2,4-dichloro-6-(1,1,3,3-tetramethylbutylamino)-1,3,5-triazine obtained in Example 1 was replaced by those obtained in Example 2(a) and (b), respectively, to obtain 164.7 g of solid resin, which was then crushed.

At the end of addition of the solution, total amount of the xylene distilled off was 87 g, which means that, at the end of the addition, xylene content in the mixture was 0.36 weight part per 1 weight part of the concentrated mass.

Yield 91 %

Number average molecular weight 4400

n = 6.7

Example 3

(a) Preparation of intermediate

Example 1 (a) was repeated, except that the reaction temperature (65°C) and amount of 2,2,6,6-tetramethyl-4-piperidone (94.6 g, 0.609 mole) were changed to 59°C and 89.6 g (0.577 mole), respectively to obtain 115.3 g of a concentrated mass. (The molar ratio of 2,2,6,6-tetramethyl-4-piperidone to hexamethylenediamine is 1.8/1.) The mass thus obtained contained 80.3 % of N,N'-bis(2,2,6,6-tetramethyl-4-piperidyl)hexamethylenediamine and 19.4 % of N-(2,2,6,6-tetramethyl-4-piperidyl)hexamethylenediamine.

(b) Preparation of dichlorotriazine

Into flask, 20 g of water, 50.0 g (0.271 mole) of cyanuric chloride and 125 g of a mixed xylene were charged and 35.9 g (0.278 mole) of 1,1,3,3-tetramethylbutylamine was slowly and dropwise added into the mixture while keeping the temperature at 8 - 10°C. Then, 55.5 g of 20 % aqueous hydroxide solution was slowly and dropwise added into the mixture while keeping the temperature at 8 - 10 °C. After completion of the reaction, oil and water phase were separated and water phase was removed off to obtain a xylene solution of 2,4-dichloro-6-(1,1,3,3-tetramethylbutylamino)-1,3,5-triazine.

(c) Preparation of polyaminotriazine

Example 1(c) was repeated, except that the concentrated mass and the xylene solution of 2,4-dichloro-6-(1,1,3,3-tetramethylbutylamino)-1,3,5-triazine obtained in Example 1 were replaced by those obtained in Example 3(a) and (b), respectively, and the amount of sodium hydroxide powder (28.4 g, 0.71 mole) was changed to 27.2 g (0.68 mole) to obtain 161.7 g of a solid resin, which was then crushed.

At the end of addition of the xylene solution, total amount of the xylene distilled off was 85 g, which means that, at the end of the addition, xylene content in the mixture was 0.35 weight part per 1 weight part of the concentrated mass.

Yield 93 %

Number average molecular weight 3700

n = 5.5

Example 4

(a) Preparation of intermediate

Example 1(a) was repeated, except that reaction temperature (65 °C) and amount of 2,2,6,6-tetramethyl-4-piperidone (94.6 g, 0.609 mole) were changed to 60°C and 93.6 g (0.603 mole), respectively to obtain 119.4 g of a concentrated mass. (The molar ratio of 2,2,6,6-tetramethyl-4-piperidone to hexamethylenediamine is 1.88/1.) The mass thus obtained contained 94.6 % of N,N'-bis(2,2,6,6-tetramethyl-piperidyl)hexamethylenediamine and 4.8 % of N-(2,2,6,6-tetramethyl-4-piperidyl)hexamethylenediamine.

(b) Preparation of dichlorotriazine

Example 1(b) was repeated to obtain a xylene solution of 2,4-dichloro-6-(1,1,3,3-tetramethylbutylamino)-1,3,5-triazine.

(c) Preparation of polyaminotriazine

Into an autoclave, whole of the concentrated mass obtained in Example 4(a) and 56 g of 50% aqueous sodium hydroxide solution were charged, and the inner temperature was raised to 180°C. Then, into the mixture, the xylene solution of 2,4-dichloro-6-(1,1,3,3-tetramethylbutylamino)-1,3,5-triazine obtained in Example 4(b) was dropwise added over 4 hours. After start of the dropwise addition, raise of inner temperature was observed and, after a while, raise of inner pressure was also observed. After the end of the addition of the solution, the reaction temperature was kept at 180°C for 5 hours. After completion of the reaction, water phase containing sodium chloride solution generated in the reaction was removed. Resulting xylene solution was filtered to remove by-products and concentrated. The resulting product was cooled to obtain 165.6 g of solid resin, which was then crushed.

Yield 92 %

Number average molecular weight 4700

n = 7.2

Example 5

(a) Preparation of intermediate

Into a flask, 92.2 g (0.594 mole) of 2,2,6,6-tetramethyl-4-piperidone and 37.3 g (0.0.321 mole) of hexamethylenediamine were charged and the mixture was kept at 50 - 60°C for one hour and then water was removed at 60°C, 30 Torr for 3 hours. The resulting reaction mass and 0.55 g of 5 % platinum/carbon were charged in an autoclave and, then, the mixture was kept at 90°C hydrogen pressure of 5 atom for 4 hours. (The molar ratio of 2,2,6,6-tetramethyl-4-piperidone to hexamethylenediamine is 1.85/1.) After completion of the reaction, catalyst was filtered off to obtain 121.9 g of reaction product. The mass thus obtained contained 92.2 % of N,N'-bis(2,2,6,6-tetramethyl-4-piperidyl)hexamethylenediamine and 7.5 % of N-(2,2,6,6-tetramethyl-4-piperidyl) hexamethylenediamine.

(c) Preparation of polyaminotriazine

Example 4(c) was repeated, except that the concentrated mass obtained in Example 4(a) was replaced by whole of reaction product obtained in Example 5(a) to obtain 162.3 g of solid resin, which was then crushed.

Yield 91 %

Number average molecular weight 4800

n = 7.0

Example 6

(a) Preparation of intermediate

Example 5(a) was repeated, except that the amount of 2,2,6,6-tetramethyl-4-piperidone of (92.2 g, 0.594 mole) were changed to 94.7 g (0.610 mole) to obtain 121.0 g of reaction product. (The molar ratio of 2,2,6,6-tetramethyl-4-piperidone to hexamethylenediamine is 1.9/1.) The mass thus obtained contained 95.1 % of N,N'-bis(2,2,6,6-tetramethyl-4-piperidyl) hexamethylenediamine and 4.5 % of N-(2,2,6,6-tetramethyl-4-piperidyl)hexamethylenediamine.

(b) Preparation of dichlorotriazine

Example 1(b) was repeated to obtain xylene solution of 2,4-dichloro-6-(1,1,3,3-tetramethylbutylamino)-1,3,5-triazine.

c) Preparation of polyaminotriazine

Example 1(c) was repeated, except that the concentrated mass and the xylene solution of 2,4-dichloro-6-(1,1,3,3-tetramethylbutylamino)-1,3,5-triazine obtained in Example 1 were replaced by those obtained in Example 6(a) and (b), respectively to obtain 168.4 g of solid resin, which was then crushed.

At the end of addition of the xylene solution, total amount of the xylene distilled off was 89 g, which means that, at the end of the addition, xylene content in the mixture was 0.34 weight part per 1 weight part of the concentrated mass.

Yield 93 %

Number average molecular weight 4700

n = 7.2

Example 7

(a) Preparation of intermediate

Example 1(a) was repeated, except that reaction temperature (65 °C ), and amount of 2,2,6,6-tetramethyl-4-piperidone (94.6 g, 0.609 mole) were changed to 61 °C and 104.6 g (0.674 mole), respectively to obtain 129.1 g of concentrated mass. (The molar ratio of 2,2,6,6-tetramethyl-4-piperidone to hexamethylenediamine is 2.1/1.) The mass thus obtained contained 97.6 % of N,N'-bis(2,2,6,6-tetramethyl-4-piperidyl)hexamethylenediamine and 2.0 % of 2,2,6,6-tetramethyl-4-piperidone, but N-(2,2,6,6-tetramethyl-4-piperidyl)hexamethylenediamine was not observed.

(b) Preparation of dichlorotriazine

Example 1(b) was repeated, except that amounts of cyanuric chloride, mixed xylene, 1,1,3,3-tetramethylbutylamine and 20 % aqueous hydroxide solution were changed to 53.0 g (0.287 mole), 132 g, 38.0 g (0.294 mole) and 58.8 g, respectively to obtain a xylene solution of 2,4-dichloro-6-(1,1,3,3-tetramethylbutylamino)-1,3,5-triazine.

(c) Preparation of polyaminotriazine

Example 1(c) was repeated, except that the concentrated mass and the xylene solution of 2,4-dichloro-6-(1,1,3,3-tetramethylbutylamino)-1,3,5-triazine obtained in Example 1 were replaced by those obtained in Example 7(a) and (b), respectively, and amount of sodium hydroxide powder (28.4 g, 0.71 mole) was changed to 28.8 g (0.72 mole) to obtain 175.9 g of solid resin, which was then crushed.

At the end of addition of the xylene solution, total amount of the xylene distilled off was 88 g, which means that, at the end of the addition, xylene content in the mixture was 0.34 weight part per 1 weight part of the concentrated mass.

Yield 92 %
Number average molecular weight 7200
n = 11.4

Reference example 1

[Degree of coloration of polyaminotriazine]

Light transmittance of solutions of polyaminotriazin es obtained in Examples 1 - 7 were measured, according to measuring conditions mentioned below.

Higher the transmittance is, more colorless and transparent the polyaminotriazine is.

[Measuring conditions]

(1) Preparation of the samples

1.0 g of polyaminotriazines obtained in each of Examples 1 - 7 was dissolved in 10 ml of toluene and was filtered with a membrane filter.

(2) Apparatus used for the measuring

U-3400 type Spectrometer
(manufactured by Hitachi Co., Ltd.)
Measuring wave length : 300 - 600 nm
Scan speed : 120 nm/min.

Table 1

| Example No. | Light transmittance (%) | | |
|---|---|---|---|
| | 350 nm | 400 nm | 450 nm |
| 1 | 38.06 | 73.01 | 89.24 |
| 2 | 68.57 | 89.15 | 95.52 |
| 3 | 62.75 | 86.70 | 95.46 |
| 4 | 26.90 | 78.76 | 91.90 |
| 5 | 66.24 | 88.72 | 94.58 |
| 6 | 53.91 | 89.79 | 98.23 |
| 7 | 16.10 | 55.65 | 83.86 |

Reference example 2

Using linear low density polyethylene (L-LDPE), compounding ingredients shown below were dry-blended.

| Compounding composition | |
|---|---|
| Unstabilized L-LDPE | 100 parts |
| Calcium stearate | 0.1 part |
| * 1 | 0.1 part |
| * 2 | 0.08part |
| Test stabilizer * 3 | 0.1 part |

* 1 : n-Octadecyl 3-(3,5-di-tert-butyl-4-hydroxyphenyl) propionate
* 2 : Tetrakis (2,4-di-tert-butylphenyl)-4,4'-biphenylene phosphonite
* 3 : obtained in Example 6 or 10

Each of the dry-blended mixture was melt-kneaded by using a 30 mm∅ monoaxial extruder at 210°C and injected from T-die to form a film with thickness of 20±2 μm. This film was punched to obtain a test piece. The test piece was put in a sunshine weather-O-meter and irradiated with light under the following conditions : light source: carbon arc, black panel temperature : 83 °C, water spray time of 18 minutes per 120 minutes cycle. When stabilizer obtained in Example 6 is used, it took 600 hours until the test piece became unable to be stretched more than 50 %. When stabilizer obtained in Example 10 is used, it took 610 hours until the test piece

became unable to be stretched more than 50 %. The result shows that the polyaminotriazine obtained in Example 6, wherein unrefined crude product (A) was used as diamine (II), has sufficient stabilizing effect as a stabilizer, which is almost same to the stabilizing effect of the polyaminotriazin e obtained in Example 10, wherein refined diamine (II) was used.

Example 8

Into 500 ml four necked flask equipped with a dean stark trap, 84.5 g (0.214 mole) of N,N'-bis(2,2,6,6-tetramethyl-4-piperidyl)hexamethylenediamine and 20.0 g (0.5 mole) of sodium hydroxide powder were charged, and inner temperature was raised to 160°C. Then, into the mixture, solution containing 55.4 g (0.20 mole) of 2,4-dichloro-6-(1,1,3,3-tetramethylbutylamino)-1,3,5-triazine in 92 g of mixed xylene having the boiling point of 138 - 141°C was dropwise added over 4 hours.

After start of dropwise addition, reflux of water generated in the reaction was observed, the refluxing water being distilled off, and, after a while, reflux of xylene also started. Keeping the dropwise addition for a while, the reaction temperature started lowering. In order to keep the reaction temperature at 160°C or higher, a part of the refluxing xylene was distilled off. At the end of addition of the solution, total amount of the xylene distilled off was 62 g, which means that, at the end of the addition, xylene content in the mixture was 0.36 weight part per 1 weight part of the diamine. After the end of the addition of the solution, the reaction temperature was kept at 160 °C for 5 hours while distilling off water generated in the reaction. After the completion of reaction, water was added to the reaction mass in order to dissolve sodium chloride generated in the reaction and removed off. Resulting xylene solution was filtered to remove by-products and concentrated. The resulting product was cooled to obtain solid resin, which was then crushed.

Yield 94 %

(Hereinafter, Yields are based on the amount of the diamine of formula (II))

Number average molecular weight 6500

n = 10.2

Example 9

Same procedures in Example 8 were repeated except that the reaction temperature was changed to 170°C.

At the end of addition of the solution, total amount of the xylene distilled off was 67 g, which means that, at the end of the addition, xylene content in the mixture was 0.29 weight part per 1 weight part of the diamine.

Yield 94 %

Number average molecular weight 5700

n = 8.9

Example 10

Same procedures in Example 8 were repeated except that amount of N,N'-bis(2,2,6,6-tetramethyl-4-piperidyl) hexamethylenediamine was changed to 86.8 g (0.22 mole).

Yield 93 %

Number average molecular weight 4500

n = 6.9

Example 11

Same procedures in Example 8 were repeated except that amount of N,N'-bis(2,2,6,6-tetramethyl-4-piperidyl) hexamethylenediamine was changed to 82.9 g (0.21 mole).

Yield 95 %

Number average molecular weight 6900

n = 10.9

Example 12

Same procedures in Example 8 were repeated except that 84.5 g of N,N'-bis(2,2,6,6-tetramethyl-4-piperidyl) hexamethylenediamine was replaced by 84.5 g of a mixture of N-(2,2,6,6-tetramethyl-4-piperidyl)hexamethylenediamine and N,N'-bis(2,2,6,6-tetramethyl-4-piperidyl)hexamethylenediamine (according to an areametric analysis measured by gas chromatography, the mixture contained 12 % of N-(2,2,6,6-tetramethyl-4-piperidyl)hexamethylenediamine.).

Yield 90 %

Number average molecular weight 4400

n = 6.7

Example 13

Same procedures in Example 8 were repeated except that solution containing 55.4 g (0.20 mole) of 2,4-dichloro-6-(1,1,3,3-tetramethylbutylamino)-1,3,5-triazine in 92 g the of mixed xylene was replaced by solution containing 47.0 g (0.20 mole) of 2,4-dichloro-6-morpholino-1,3,5-triazine in 92 g of the mixed xylene.

Yield 91 %

Number average molecular weight 2500

n = 3.7

Comparative example 1

Same procedures in Example 9 were repeated except that xylene was replaced by 110 g of o-dichlorobenzene and that distilling off the o-dichlorobenzene was not conducted. (But, distilling off the water was conducted.)

Yield 88 %

Number average molecular weight 5400

n = 8.3

Comparative example 2

Into 1 L four necked flask, 84.5 g (0.214 mole) of N,N'-bis(2,2,6,6-tetramethyl-4-piperidyl)hexamethylenediamine , 20.0 g (0.5 mole) of sodium hydroxide powder and solution containing 55.4 g (0.20 mole) of 2,4-dichloro-6-(1,1,3,3-tetramethylbutylamino)-1,3,5-triazine in 300 g of toluene were charged. Then, the inner temperature was raised to start reflux and kept the refluxing condition for 16 hours. After completion of the reaction, by-products and salts generated in the reaction were removed by filtration. By washing with water, concentration and cooling, a solid resin was obtained. Then, the solid resin was crushed.

Yield 72 %

Number average molecular weight 2800

n = 4.0

Comparative example 3

Into a 500 ml autoclave made of SUS 316 L, 86.8 g (0.22 mole) of N,N'-bis(2,2,6,6-tetramethyl-4-piperidyl) hexamethylenediamine and 37 g of 48 % aqueous sodium hydroxide solution were charged, and inner temperature was raised to 180 °C. Then, into the mixture, solution containing 55.4 g (0.20 mole) of 2,4-dichloro-6-(1,1,3,3-tetramethylbutylamino)-1,3,5-triazine in 98 g of xylene was dropwise added over 4 hours. After the end of the addition of the solution, the reaction temperature was kept at 185°C for 5 hours. Then, after phase separation, washing with water and filtration to remove by-products, the resulting product was concentrated and cooled to obtain solid resin. Then, the solid resin was crushed. The autoclave was so eroded that it must be disused.

Yield 89 %

Number average molecular weight 4800

n = 7.3

## Claims

1. A process for preparing a polyaminotriazine of the formula (I):

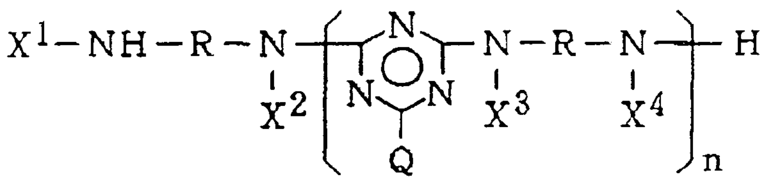

(I)

wherein n is from 2 to 20;
$X^1$, $X^2$, $X^3$ and $X^4$, which are the same or different, are each hydrogen or piperidyl of the formula (Ia):

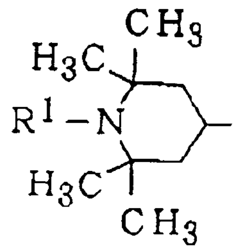

(Ia)

wherein $R^1$ is a hydrogen atom, $C_1$ to $C_{12}$ alkyl, $C_1$ to $C_{18}$ alkoxy, $C_3$ to $C_8$ alkenyl, $C_7$ to $C_{11}$ arylalkyl or $C_3$ to $C_5$ alkenyloxy, provided that 75mole% or more of $X^1$, $X^2$, $X^3$ and $X^4$ is piperidyl of formula (Ia);
R is $C_2$ to $C_{12}$ alkylene, which can be interrupted by -O- or $-NR^2-$ wherein $R^2$ is hydrogen, $C_1$ to $C_{12}$ alkyl, $C_3$ to $C_{12}$ cycloalkyl or piperidyl of the formula (Ia), or R is a divalent $C_6$ to $C_{15}$ cycloaliphatic group; and Q is $-OR^3$, $-NHR^4$ or $-NR^4R^5$
wherein $R^3$ is $C_1$ to $C_{12}$ alkyl, $C_5$ to $C_{12}$ cycloalkyl, benzyl, phenyl, tolyl or piperidyl of the formula (Ia), $R^4$ is $C_1$ to $C_{12}$ alkyl, $C_3$ to $C_{12}$ alkoxyalkyl, $C_4$ to $C_{12}$ N,N-dialkylaminoalkyl, $C_3$ to $C_5$ alkenyl, phenyl, benzyl, $C_5$ to $C_{12}$ cycloalkyl, tolyl or piperidyl of the formula (Ia), and $R^5$ is $C_1$ to $C_{12}$ alkyl or $C_5$ to $C_{12}$ cycloalkyl, or $R^4$ and $R^5$ together with the N atom to which they are bonded form a 5- or 6- membered heterocyclic ring which process comprises;

the reduction alkylamination of a tetramethylpiperidone of the formula (IIa):

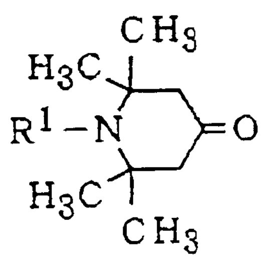

(IIa)

wherein $R^1$ is as hereinbefore defined, with a diamine represented by the formula (IIb):

$NH_2$-R-$NH_2$ (IIb)

wherein R is hereinbefore defined, in the presence of a hydrogenation catalyst;
removing the catalyst after the completion of the reaction to obtain a crude product (A);
and then, without refining the crude product (A), carrying out a polycondensation reaction between the crude product (A) and a dichlorotriazine of the formula (III):

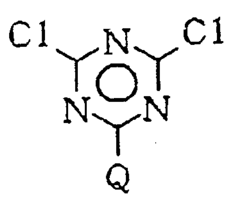

(III)

wherein Q is as hereinbefore defined, in an aromatic solvent in the presence of an inorganic base.

2. A process according to Claim 1, wherein the amount of the tetramethylpiperidone is from 1.7 to 1.95 mole per mole of the diamine.

3. A process according to Claim 1 or 2, wherein the amount of the dichlorotriazine is from 0.8 to 0.9 mole per mole of the diamine.

4. A process according to Claim 1, 2 or 3 wherein the tetramethylpiperidone and the diamine are reacted at 50-100°C in the absence of solvent while distilling off water, the product obtained is subjected to reduction alkylamination with hydrogen in the presence of platinum, palladium or Raney nickel and the catalyst is then removed to obtain crude product (A).

5. A process according to Claim 1, 2 or 3 wherein the hydrogenation catalyst is platinum, palladium or Raney nickel, the reductive alkylamination is carried out in methanol, ethanol, propanol or a mixture of the alcohol and water and the catalyst and solvent are then removed.

6. A process according to any one of the preceding claims, wherein the reduction alkylamination is carried out in the presence of 0.001 to 0.04 weight% of platinum or palladium based on the tetramethylpiperidone at a temperature from 40 to 140°C.

7. A process according to any one of Claims 1 to 5, wherein the reduction alkylamination is carried out in the presence of 0.1 to 30 weight% of Raney nickel based on the tetramethylpiperidone at a temperature from 100 to 180°C.

8. A process according to any of the preceding claims, wherein the polycondensation reaction is carried out under elevated pressure, by adding a solution of the dichlorotriazine in an aromatic solvent to a mixture of the crude product (A) and an about 50% aqueous solution of sodium hydroxide or potassium hydroxide at a temperature from 145 to 220°C.

9. A process according to any one of Claims 1 to 7, wherein the polycondensation reaction is carried out under atmospheric pressure, by adding a solution of the dichlorotriazine in an aromatic solvent to a mixture of the crude product (A) and solid sodium hydroxide or potassium hydroxide which is kept at 145-190°C while distilling off water and a part of the solvent.

10. A process according to Claim 9, wherein the amount of solvent is 0.1-1 part by weight of the crude product (A).

11. A process according to Claim 9 or 10, wherein the mixture of the crude product (A) and solid sodium hydroxide or potassium hydroxide is kept at 155-180°C.

12. A process for preparing a polyaminotriazine of formula (I) as defined in Claim 1 which comprises;
reacting a diamine of the formula (II)

$$X\text{-}NH\text{-}R\text{-}NH\text{-}X^5$$

wherein R is as defined in Claim 1, X is piperidyl of formula (Ia) as defined in Claim 1 and $X^5$ is hydrogen or piperidyl of formula (Ia) as defined in Claim 1 with a dichlorotriazine of the formula (III) as defined in Claim 1 in a mole ratio (III)/(II) of 0.83 to 0.98, provided that more than 80 mole% of the diamine of the formula (II) is that wherein $X^5$ is piperidyl of the formula (Ia), in 0.1-1 part by weight of a water-immiscible aromatic solvent per part of the diamine, using an inorganic base in a solid from, under atmospheric pressure at a temperature higher than the boiling point of the solvent while azeotropically distilling off water generated in the reaction.

13. A process according to any one of the preceding claims, wherein $R^1$ is hydrogen or methyl, R is straight chain alkylene having 2-12 carbon atoms and Q is $-NHR^4$ or $-NR^4R^5$.

14. A process according to any one of the preceding claims, wherein Q is $-NHR^4$.

15. A process according to any one of the preceding claims, wherein $R^1$ is hydrogen, R is hexamethylene and Q is 1,1,3,3-tetramethylbutylamino.

16. A process according to any one of Claims 12 to 15, wherein the amount of the solvent is 0.2-0.8 part by weight of the crude product (A) and the reaction temperature is 155-180°C.

**17.** A process according to any one of Claims 12 to 16, wherein the reaction is carried out by adding a solution of the dichlorotriazine of the formula (III) in an aromatic solvent to a mixture of the diamine of the formula (II) and sodium hydroxide or potassium hydroxide which is kept at a temperature higher than the boiling point of the solvent while distilling off water generated in the reaction and a part of the solvent.

European Patent Office

# EUROPEAN SEARCH REPORT

Application Number
EP 94 30 9681

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| Y | EP-A-0 357 223 (VYSKUMNY USTAV CHEMICKEJ TECHNOLOGIE VYSKUMNA A VYVOJOVA ORGANIZACIA)<br>* the whole document *<br>--- | 1 | C07D401/14<br>C08K5/3492 |
| Y | EP-A-0 377 324 (SLOVENSKA AKADEMIA VIED)<br>* the whole document *<br>--- | 1 | |
| Y<br><br>D | EP-A-0 093 693 (CIBA-GEIGY AG)<br>* the whole document *<br>& JP-A-58 201 820 (...)<br>--- | 1 | |
| Y | EP-A-0 065 169 (HOECHST AKTIENGESELLSCHAFT)<br>* the whole document *<br>--- | 1 | |
| Y | WO-A-83 02943 (THE B.F.GOODRICH COMPANY)<br>* the whole document *<br>--- | 1 | |
| Y<br><br><br>D | FR-A-2 333 821 (CHIMOSA CHIMICA ORGANICA S.P.A)<br>* the whole document *<br>& JP-A-52 071 486 (...)<br>----- | 1 | TECHNICAL FIELDS SEARCHED (Int.Cl.6)<br>C07D<br>C08K |

The present search report has been drawn up for all claims

| Place of search | Date of completion of the search | Examiner |
|---|---|---|
| BERLIN | 13 April 1995 | Kyriakakou, G |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)